# EUROPÄISCHE PATENTSCHRIFT

(11) **EP 4 669 246 B1**
(45) Veröffentlichungstag und Bekanntmachung des Hinweises auf die Patenterteilung: **27.05.2026**
(21) Anmeldenummer: 25724271.9
(22) Anmeldetag: 06.05.2025
(51) Int. Cl.: A61B 34/00, A61B 34/30, A61B 90/00, B25J 9/16

(54) **MEDIZINISCHES ROBOTERSYSTEM**
MEDICAL ROBOTIC SYSTEM
SYSTÈME ROBOTIQUE MÉDICAL

(30) Priorität: 07.05.2024 DE 102024112788
(43) Veröffentlichungstag der Anmeldung: 31.12.2025
(73) Patentinhaber: B. Braun New Ventures GmbH, 79110 Freiburg im Breisgau (DE)
(72) Erfinder: SARVESTANI, Amir, 79104 Freiburg (DE)
(74) Vertreter: Winter, Brandl - Partnerschaft mbB
(86) Internationale Anmeldenummer: PCT/EP2025/062326
(87) Internationale Veröffentlichungsnummer: WO 2025/233326

(56) Entgegenhaltungen:
- WO-A1-2023/126770
- DE-A1- 102021 102 274
- US-A1- 2009 192 524
- US-A1- 2017 196 453
- US-A1- 2020 253 678
- US-A1- 2021 304 637

## Beschreibung

Die vorliegende Offenbarung betrifft ein medizinisches, vorzugsweise chirurgisches, Robotersystem, mit einem Roboter, der zumindest einen beweglichen Roboterarm, vorzugsweise zum Positionieren chirurgischer Instrumente und/oder eines Visualisierungssystems, aufweist, einer (ersten) optischen Aufnahmevorrichtung, die an dem Roboterarm, vorzugsweise einem Endeffektor des Roboterarms, angebracht und eingerichtet ist, eine (erste) optische Aufnahme zu erstellen, und einer Anzeige, die eingerichtet ist, die (erste) optische Aufnahme abzubilden.

### Hintergrund der Offenbarung

Der Einsatz von medizinischen Robotersystemen ist insbesondere in der Chirurgie weit verbreitet, um einen präzisen, beispielsweise minimalinvasiven Eingriff zu unterstützen. Dabei kann ein Roboter mit zumindest einem beweglichen Roboterarm insbesondere eingesetzt werden, um chirurgische Instrumente im Raum zu positionieren und/oder einzelne Eingriffsoperationen durchzuführen. Für die Positionierung kann der bewegliche Roboterarm, insbesondere ein Endeffektor des Roboterarms, an dem ein betreffendes chirurgisches Instrument gehalten wird, im Raum bewegt werden. Ein Risiko hierbei ist, dass bei der Bewegung des Roboterarms eine Kollision zwischen dem Roboterarm bzw. dem Endeffektor und der Umgebung, wie einem medizinischen Fachpersonal, insbesondere einem Chirurgen, oder einem Patienten, auftreten kann.

Um ein solches Risiko einer Kollision zu vermindern, gibt es medizinische Robotersysteme mit einem System zur Kollisionserkennung. Eine Kollisionserkennung kann, wie es beispielsweise in der Patentschrift CN 1 10 613 511 A offenbart wird, in einem medizinischen Robotersystem, ähnlich zu einer Kollisionserkennung in Fahrzeugen, unter Verwendung von Ultrasound-Sensoren, Lidar-Sensoren, oder 3D-Punktwolken als Abstandsregelung implementiert werden. Allerdings ist eine solche Kollisionserkennung nicht sehr zuverlässig aufgrund von ständigen Veränderungen im Kollisionsraum des Roboters, zum Beispiel aufgrund von Bewegungen des Chirurgen. Eine Alternative ist die Modellierung der Umgebung des Roboters, insbesondere die Position des Patienten, mit Hilfe von Navigations- und vor-operativen Daten. Ein solches statisches Verfahren berücksichtigt aber keine Veränderungen während einer Operation am Patienten. Außerdem ermöglicht es keine Kollisionserkennung mit nicht modellierten Objekten. Aufgrund des Fehlens einer zuverlässigen Kollisionserkennung hängt somit die Sicherheit davon ab, wie vorsichtig/sorgsam der Roboter bewegt wird.

Zudem gibt es medizinische Robotersysteme, die eine an dem Roboterarm, insbesondere an dem Endeffektor, angebrachte Aufnahmevorrichtung/Kamera zum Erstellen einer optischen Aufnahme und eine Anzeige zum Abbilden der Aufnahme aufweisen, um die (nahe) Umgebung und insbesondere einen möglichen Kollisionsbereich des Roboterarms anzuzeigen. Diese Kameraüberwachung bzw. optische Anzeige der Umgebung bildet somit ein passives Kamerasystem, das dazu dient, eine Steuerung der Bewegung des Roboterarms zu ermöglichen und/oder zu vereinfachen bzw. insbesondere Kollisionen des Roboterarms durch sorgsame Steuerung und Kenntnis der nahen Umgebung zu vermeiden.

Nachteilig ist daran jedoch, dass - insbesondere wenn die Roboterarmsteuerung durch den Chirurgen erfolgt - die permanente optische Anzeige zur Umgebungsüberwachung dazu führen kann, dass der Chirurg dieser mit der Zeit immer weniger Aufmerksamkeit schenkt und somit das Ziel der Verringerung eines Kollisionsrisikos verfehlt wird. Außerdem kann es dazu führen, dass die Aufmerksamkeit des Chirurgen von dem Operationsfeld abgelenkt wird, was wiederum die Patientensicherheit gefährdet.

DE 102021102274 A1 offenbart ein chirurgisches Assistenzsystem mit einem Operationsmikroskop und einer Umgebungskamera, die an einem Roboterarm angebracht sind, wobei die Umgebungskamera mittels einer Tiefenwahrnehmung dazu verwendet werden kann, um einen Abstand zu einem Hindernis zu berechnen, wobei abhängig des Abstandes dann eine Alarmanzeige über eine Darstellungsvorrichtung angezeigt werden kann.

US 2017/196453 A1 offenbart ein System für die Augenchirurgie, das ein Operationsmikroskop aufweisen kann.

US 2009/192524 A1 offenbart eine künstliche Repräsentation eines robotischen Werkzeuges zur Anzeige für einen Benutzer eines robotischen Systems.

### Kurzbeschreibung der Offenbarung

In Anbetracht der oben beschriebenen Problematik ist es daher eine Aufgabe der Offenbarung, ein geeignetes medizinisches Robotersystem bereitzustellen, welches die (Patienten-) Sicherheit erhöht und insbesondere einen Anwender/ Benutzer, beispielsweise den Chirurgen, dabei unterstützt, eine Kollision des medizinischen Robotersystems, insbesondere mit einem Patienten, zu vermeiden.

Diese Aufgabe wird durch ein medizinisches Robotersystem nach Anspruch 1 gelöst. Vorteilhafte Ausführungsformen sind Gegenstand der abhängigen Ansprüchen und/oder nachfolgend in der Beschreibung beschrieben.

Das offenbarungsgemäße medizinische Robotersystem hat einen Roboter, der zumindest einen beweglichen Roboterarm aufweist, eine (erste) optische Aufnahmevorrichtung, die an dem Roboterarm angebracht und eingerichtet ist, eine (erste) optische Aufnahme zu erstellen, eine Anzeige, die eingerichtet ist, die (erste) optische Aufnahme abzubilden, und eine Auslösesteuerung, die eingerichtet ist, ein Abbilden der (ersten) optischen Aufnahme auf der Anzeige in Abhängigkeit einer Bewegung des Roboterarms zu steuern, um (somit) einen Anwender (des medizinischen Robotersystems) auf die Bewegung des Roboterarms aufmerksam zu machen.

Die Vorteile der Offenbarung bestehen darin, dass das medizinisches Robotersystem das Risiko einer Kollision mindert, indem die erste optische Aufnahme in Abhängigkeit der Bewegung des Roboterarms auf der Anzeige angezeigt wird. Wenn sich beispielsweise der Roboterarm zu bewegen beginnt, kann dies eine Veränderung der Anzeige bzw. des Abbildens der ersten optischen Aufnahme auslösen. Somit wird ein Anwender, beispielsweise ein Chirurg, auf die Bewegung aufmerksam gemacht. Dies reduziert die Kollisionsgefahr und erhöht die Patientensicherheit. Wenn die erste optische Aufnahme zum Beispiel nur während einer Bewegung des Roboterarms angezeigt wird und/oder dabei hervorgehoben wird und sonst nicht, wird ein Anwender bei Bewegungslosigkeit des Roboterarms nicht durch die erste optische Aufnahme von der Arbeit abgelenkt. Dies erhöht ebenfalls die Patientensicherheit. In anderen Worten wird bevorzugt die Aufmerksamkeit eines Anwenders nur dann auf die erste optische Aufnahme gelenkt, wenn dies nötig ist bzw. die Sicherheit erhöht. Dabei unterstützt das Robotersystem vorzugsweise den Anwender eine Kollision zu verhindern, indem die Umgebung des Roboters als erste optische Aufnahme aufgenommen und auf der Anzeige abgebildet wird.

Mit anderen Worten betrifft die vorliegende Offenbarung ein medizinisches, vorzugsweise chirurgisches Robotersystem, das einen (in einer Operationsumgebung) beweglichen Roboterarm, eine am Roboterarm montierte (und sich entsprechend mit dem Roboterarm mitbewegende) Aufnahmevorrichtung/Kamera zur insbesondere weitwinklingen Erfassung der Operationsumgebung und eine Anzeige zur Darstellung der mit der Aufnahmevorrichtung erfassten Operationsumgebung (d.h. der optischen Aufnahme). Dabei ist die Anzeige konfiguriert (bzw. das medizinische Robotersystem weist eine Auslösesteuerung/Aufnahmevorrichtungssteuerung/Anzeigensteuerung auf, die eingerichtet ist), um die mit der Aufnahmevorrichtung erfasste Operationsumgebung in Abhängigkeit einer Bewegung des Roboterarms auf der Anzeige darzustellen/abzubilden/anzuzeigen.

Es ist also ein Kerngedanke der Offenbarung, das Abbilden bzw. die Darstellungsweise bzw. Abbildungsweise der optischen Aufnahme in Abhängigkeit einer Bewegung des Roboterarms zu steuern. Das heißt, die Darstellungsweise wird also abhängig von der Roboterarmbewegung verändert, um durch diese Veränderung einen Anwender bzw. Benutzer des Robotersystems auf die Bewegung aufmerksam zu machen. Es versteht sich, dass sich aufgrund der Bewegung die optische Aufnahme selbst verändern kann, wenn die optische Aufnahmevorrichtung bewegt wird. Es ist daher nicht die Idee, die optische Aufnahme bzw. das Bild an sich/ selbst zu verändern, also z.B. einen Bildparameter (z.B. den Bildausschnitt, Zoom etc.) anzupassen, sondern (darüber hinaus) das Abbilden bzw. die Darstellungsweise zu verändern, um die Aufmerksamkeit dadurch auf die Roboterarmbewegung zu lenken. Mit anderen Worten wird unter einer Steuerung des Abbildens der optischen Aufnahme auf der Anzeige eine bildparameterunabhängige bzw. bildinhaltunabhängige Veränderung der Darstellungsweise verstanden.

Erfindungsgemäß ist die Anzeige so eingerichtet bzw. gesteuert, um die Operationsumgebung nur dann anzuzeigen, wenn sich der Roboterarm bewegt. Das heißt, dass die Operationsumgebung nur während der Bewegung angezeigt wird bzw. nicht vor dem Beginn der Bewegung und nach dem Ende der Bewegung angezeigt wird d.h., wenn sich der Roboterarm noch nicht bewegt und/oder nicht mehr bewegt. Alternativ oder zusätzlich kann die Auslösesteuerung z.B. eingerichtet sein, die optische Aufnahme bei einer Bewegung des Roboterarms optisch (besonders) hervorzuheben (wie nachfolgend ausführlicher beschrieben).

In anderen Worten ist die Auslösesteuerung bevorzugt dazu eingerichtet, die optische Aufnahme auf der Anzeige in Abhängigkeit der Bewegung des Roboterarms hervorzuheben und/oder die optische Aufnahme auf der Anzeige nur anzuzeigen, wenn sich der Roboterarm bewegt.

Die Auslösesteuerung ist also eingerichtet, ein Abbilden bzw. eine Darstellungsweise der optischen Aufnahme auf der Anzeige in Abhängigkeit einer Bewegung des Roboterarms zu steuern bzw. zu verändern, und nicht (nur) einen Bildparameter (der optischen Aufnahme) in Abhängigkeit einer Bewegung des Roboterarms zu steuern bzw. zu verändern.

Bevorzugt handelt es sich bei dem medizinischen Robotersystem um ein medizinisches und insbesondere chirurgisches Robotersystem. In anderen Worten ist das Einsatzgebiet des Robotersystems bevorzugt die Chirurgie. Die Offenbarung ist aber nicht darauf beschränkt. Das medizinische Robotersystem kann auch in anderen Fachbereichen eingesetzt werden, beispielsweise der Dermatologie oder Ophthalmologie.

Bevorzugt ist der Roboter ein sechsachsiger Roboter mit sechs Freiheitsgraden, besonders bevorzugt ein sechsachsiger Gelenkarmroboter oder sechsachsiger Knickarmroboter. Bevorzugt hat der Roboter einen Roboterarm, der geeignet und/oder eingerichtet ist, chirurgische Instrumente und/oder ein Visualisierungssystem zu positionieren. Dann kann der Roboter einen Chirurgen bei einer Operation unterstützen. Bevorzugt ist ein solches Visualisierungssystem beispielsweise ein chirurgisches Mikroskop und/oder ein Endoskop. Besonders bevorzugt ist es eine optische Aufnahmevorrichtung, vorzugsweise eine der hierin genannten. In anderen Worten kann es sich bei dem Visualisierungssystem um eine optische Aufnahmevorrichtung handeln.

Bevorzugt hat der Roboter einen Endeffektor, d.h. eine Vorrichtung an einem (freien) Ende des Roboterarms bzw. ein letztes/endseitiges kinematisches Roboterarmelement, die bzw. das dazu eingerichtet ist, mit der Umgebung und/oder dem Patienten zu interagieren. Der Endeffektor kann zum Beispiel zumindest ein Greifer oder eine Halterung sein/ aufweisen, welche(r) beispielsweise ein Visualisierungssystem und/oder chirurgische Instrumente aufnehmen und/oder halten kann. Der Endeffektor kann auch selbst ein Visualisierungssystem und/oder eine optische Aufnahmevorrichtung sein, beispielsweise eine der hierin genannten.

Unter der ersten optischen Aufnahmevorrichtung ist bevorzugt eine Kamera zu verstehen. Bevorzugt ist die erste optische Aufnahmevorrichtung am Roboterarm, besonders bevorzugt am Endeffektor, angebracht. Bevorzugt ist die erste optische Aufnahme weitwinklig bzw. eine Weitwinkelaufnahme. Bevorzugt ist also die erste optische Aufnahmevorrichtung eingerichtet, Weitwinkelaufnahmen zu machen. Dies hat den Vorteil, dass ein besonders großer Bildwinkel aufgenommen werden kann, wodurch die Aufnahme, insbesondere auch bei kürzerem Abstand zwischen der Aufnahmevorrichtung und dem zu erfassenden Aufnahmebereich, einen besseren/größeren Überblick über die aufgenommene Szene geben kann.

Bevorzugt ist die optische Aufnahmevorrichtung also extrakorporal. Bevorzugt ist die optische Aufnahme also eine (Übersichts-) Aufnahme, die vorzugsweise einem Anwender einen (möglichst guten bzw. großen) Überblick über ein Operationsfeld bzw. -gebiet ermöglicht. Bevorzugt ist die optische Aufnahmevorrichtung (daher) kein Endoskop.

Wie voranstehend beschrieben wird das Robotersystem bevorzugt in der Chirurgie eingesetzt/angewendet und unterstützt dabei einen Chirurgen. Bevorzugt ist (dazu) die erste optische Aufnahmevorrichtung auf ein Operationsfeld ausgerichtet und/oder fokussiert. Das heißt, bevorzugt ist die erste optische Aufnahmevorrichtung zum Beispiel auf einen Operationstisch und einen sich darauf befindenden Patienten (und dessen Umgebung) ausgerichtet und/oder fokussiert. Die erste optische Aufnahme ist bevorzugt eine Aufnahme von der oder von Teilen der (direkten) Umgebung des Roboters. Besonders bevorzugt ist die erste optische Aufnahme eine Aufnahme von dem Operationsfeld, insbesondere von einem Patienten, der operiert wird/ werden soll und/oder zumindest von dem Körperteil oder dem Körperabschnitt, an dem bzw. das/der operiert wird oder werden soll. Die Offenbarung ist aber nicht darauf beschränkt. Insbesondere bei Einsätzen des medizinischen Robotersystems in anderen medizinischen, nicht-operativen Fachbereichen kann beispielsweise die erste optische Aufnahmevorrichtung auf einen Bereich, beispielsweise einen Behandlungs-/Untersuchungsbereich, gerichtet sein und die erste optische Aufnahme kann dann entsprechend eine Aufnahme von diesem Bereich sein.

Ein Abbilden kann als ein (visuelles) Anzeigen und/oder Darstellen verstanden werden.

Es ist zu verstehen, dass die Anzeige einteilig oder auch mehrteilig sein kann. Die Anzeige kann also beispielsweise ein Bildschirm sein, kann aber auch mehrere Bildschirme aufweisen, die (gemeinsam) das Gleiche oder Unterschiedliches abbilden.

Bevorzugt ist das Robotersystem und/oder die Anzeige und/oder die erste optische Aufnahmevorrichtung eingerichtet, die erste optische Aufnahme als Liveübertragung bzw. Livestream bzw. Echtzeitübertragung auf der Anzeige abzubilden. In anderen Worten wird also bevorzugt, wenn die Anzeige die erste optische Aufnahme abbildet, das angezeigt, was zu (nahezu) diesem Zeitpunkt die erste optische Aufnahmevorrichtung sieht und/oder als erste optische Aufnahme aufnimmt.

Bevorzugt ist die Anzeige eingerichtet, mehrere Bilder, Videos, optische Aufnahmen usw. gleichzeitig auf verschiedene Weisen, zum Beispiel nebeneinander, ineinander als Bild im Bild usw., anzuzeigen.

Eine Bewegung des Roboterarms kann beispielweise aus einer Steuerung/ Regelung, einem Bewegungsbefehl, der beispielweise von einem Anwender über einen Joy-Stick eingegeben wird, oder daraus resultieren, dass ein Anwender den Roboterarm anfasst und bewegt.

Die Auslösesteuerung ist eingerichtet, das Abbilden der ersten optischen Aufnahme auf der Anzeige in Abhängigkeit eines Startens der Bewegung des Roboterarms, besonders bevorzugt mit Beginn der Bewegung des Roboterarms, zu beginnen. Es kann zum Beispiel dazu die erste optische Aufnahmevorrichtung (erst) jetzt eingeschalten oder aus einem Stand-by-Modus aktiv werden. Es kann auch alternativ oder zusätzlich dazu (erst) jetzt eine (Daten-) Übertragung der ersten optischen Aufnahme an die Anzeige erfolgen und/oder die Anzeige (erst) jetzt eingeschalten oder aus einem Stand-by-Modus aktiv werden. Es kann dazu zum Beispiel auch die Anzeige zunächst auch etwas Anderes, zum Beispiel eine andere Aufnahme oder ein Standbild abbilden, welches dann in Abhängigkeit eines Startens der Bewegung des Roboterarms, besonders bevorzugt mit Beginn der Bewegung des Roboterarms, durch die erste optische Aufnahme ersetzt oder ergänzt wird.

Die Auslösesteuerung ist zusätzlich dazu eingerichtet, das Abbilden der ersten optischen Aufnahme auf der Anzeige in Abhängigkeit eines Anhaltens/ Stoppens der Bewegung des Roboterarms, besonders bevorzugt mit Beendigung der Bewegung des Roboterarms, zu beenden. Dazu kann zum Beispiel die erste optische Aufnahmevorrichtung ausgeschalten oder in einen Stand-by-Modus versetzt werden. Es kann auch alternativ oder zusätzlich dazu die (Daten-) Übertragung der ersten optischen Aufnahme an die Anzeige unterbunden werden und/oder die Anzeige ausgeschalten oder in einen Stand-by-Modus versetzt werden. Die Anzeige kann dazu auch etwas anderes als die erste optische Aufnahme abbilden, beispielsweise ein Standbild oder (nur noch, ohne die erste optische Aufnahme) zumindest eine andere Aufnahme.

Die Auslösesteuerung kann (auch) eingerichtet sein, die Abbildungsweise der ersten optischen Aufnahme auf der Anzeige in Abhängigkeit des Startens der Bewegung des Roboterarms, besonders bevorzugt mit Beginn der Bewegung des Roboterarms, zu verändern. Bevorzugt kann die erste optische Aufnahme optisch hervorgehoben werden, zum Beispiel mit einer Markierung oder einem Hinweis, indem sie vergrößert wird oder größer/ am größten abgebildet wird, im Vordergrund abgebildet wird, und/oder auf eine andere Weise, wobei die Abbildung/ Abbildungsweise verändert wird und die Aufmerksamkeit stärker darauf gelenkt wird. So kann zum Beispiel in Abhängigkeit eines Startens der Bewegung des Roboterarms, besonders bevorzugt mit Beginn der Bewegung des Roboterarms, die erste optische Aufnahme (jetzt) größer, im Vordergrund, und/oder mit einer Markierung oder ähnlichem auf der Anzeige abgebildet werden.

Die Auslösesteuerung kann (dann) (auch) eingerichtet sein, die Abbildungsweise der ersten optischen Aufnahme auf der Anzeige in Abhängigkeit des Anhaltens/ Stoppens der Bewegung des Roboterarms, besonders bevorzugt mit Beendigung der Bewegung des Roboterarms, zu verändern. Bevorzugt wird eine Hervorhebung der ersten optischen Aufnahme beendet/ rückgängig gemacht.

Bevorzugt löst also eine Bewegung oder ein Beginn einer Bewegung des Roboterarms (aus vorherigem Stillstand/ Ruhe/ Bewegungslosigkeit) einen Beginn eines Abbildens oder ein andersartiges/ verändertes Abbilden der ersten optischen Aufnahme auf der Anzeige aus. Bevorzugt löst also eine Beendigung einer Bewegung des Roboterarms (Stillstand nach vorheriger Bewegung) ein Beenden des Abbildens oder des andersartigen Abbildens der ersten optischen Aufnahme auf der Anzeige aus. Bevorzugt wird während einer Bewegung des Roboterarms die erste optische Aufnahme auf der Anzeige abgebildet und sonst (bei keiner Bewegung) nicht, oder zum Beispiel während einer Bewegung die erste optische Aufnahme auf der Anzeige anders abgebildet als sonst.

Die Auslösesteuerung kann eingerichtet sein, die Abbildungsweise der ersten optischen Aufnahme auf der Anzeige, vorzugsweise wie voranstehend beschrieben, in Abhängigkeit der Bewegung des Roboterarms zu steuern/ zu verändern. Beispielsweise kann die Abbildungsweise der ersten optischen Aufnahme auf der Anzeige abhängig eines sich aufgrund der Bewegung verändernden Abstandes beispielsweise zu einem anderen Objekt, einem Patienten, oder Anwender des Robotersystems im Kollisionsraum, einer aufgrund der Bewegung drohenden Kollision, vorzugsweise wie nachfolgend beschrieben, einer Geschwindigkeit und/oder einer Beschleunigung des Roboterarms, vorzugsweise des Endeffektors und/oder daran angebrachten Vorrichtungen, gesteuert/ verändert werden. Zum Beispiel kann die erste optische Aufnahme desto stärker hervorgehoben werden, vorzugsweise wie voranstehend beschrieben, je geringer ein Abstand, je wahrscheinlicher eine Kollision (droht), je größer eine Geschwindigkeit und/oder je größer eine Beschleunigung wird/ ist. Es kann auch eine Skala, die vorzugsweise in oder mit der ersten optischen Aufnahme auf der Anzeige abgebildet wird, zum Beispiel einen Abstand, eine Kollisionsgefahr, eine Geschwindigkeit oder eine Beschleunigung visualisieren und sich abhängig davon/ von der Bewegung ändern und somit die erste optische Aufnahme hervorheben.

Eine Bewegung führt also bevorzugt zu einer Veränderung der Abbildung (der ersten optischen Aufnahme) auf der Anzeige, wobei die Auslösesteuerung dazu eingerichtet ist, die Anzeige (entsprechend) zu steuern.

Eine (solche) Veränderung auf der Anzeige/ ein solches Abbilden auf der Anzeige kann dann für einen Anwender bemerkbar sein und die Aufmerksamkeit darauf lenken. Somit wird der Anwender also bevorzugt darauf aufmerksam gemacht, dass sich der Roboterarm bewegt. Dies kann die Sicherheit erhöhen und das Risiko einer Kollision mindern.

Außerdem kann der Anwender bevorzugt mit der ersten Aufnahme sozusagen die (sich bei Bewegung verändernde) Sicht/Perspektive der ersten optischen Aufnahmevorrichtung, vorzugsweise vom Endeffektor aus, auf zum Beispiel den Patienten sehen, und somit einen (sich bei Bewegung möglicherweise verändernden) Abstand einschätzen. Dies kann ebenfalls die Sicherheit erhöhen und das Risiko einer Kollision mindern.

Bevorzugt wird die erste optische Aufnahme also nicht auf der Anzeige oder nicht hervorgehoben abgebildet, wenn sich der Roboterarm auch nicht bewegt, somit kann sich der Anwender dann zum Beispiel auf die Operation und/oder sich zum Beispiel auf eine andere optische Aufnahme, die auf der Anzeige abgebildet wird, konzentrieren und wird nicht durch die erste optische Aufnahme abgelenkt. Dies kann ebenfalls die Patientensicherheit erhöhen.

Die erste optische Aufnahmevorrichtung kann eine 2D- oder eine 3D-Kamera sein.

Bevorzugt ist die erste optische Aufnahmevorrichtung eingerichtet, 3D-Körper und/oder 3D-Oberflächen, vorzugsweise mit/ als Stereo-Rekonstruktion, Punktwolken und/oder Lidar, vorzugsweise als die erste optische Aufnahme, zu erfassen. Bevorzugt ist die Anzeige dementsprechend eingerichtet, vorzugsweise dreidimensional, solche Körper und/oder Oberflächen (ggfls. weiterverarbeitet) abzubilden. Dadurch kann eine bessere räumliche Vorstellung vermittelt werden, insbesondere kann somit dann die Tiefe/ Entfernungen besser von einem Anwender eingeschätzt werden. Es kann auch das Robotersystem Abstände oder Entfernungen zur Kollisionsvermeidung berechnen.

Bevorzugt ist das Robotersystem eingerichtet, aufgrund der erfassten 3D-Oberflächen eine drohende Kollision (basierend auf der Bewegung des Roboterarms) zu erkennen und entsprechende Maßnahmen zu ergreifen. Eine drohende Kollision kann zum Beispiel auf Basis der erfassten 3D-Oberflächen und/oder einer Bewegungssteuerung des Roboterarms erkannt werden. Es kann auch ein Mindestabstand und/oder eine Höchstgeschwindigkeit (vorzugsweise abstandsabhängig) und/oder eine Höchstbeschleunigung (vorzugsweise abstands- und/oder geschwindigkeitsabhängig) als Grenzwert, beispielsweise (im Arbeitsraum) für den Endeffektor relativ zu den/ zu bestimmten erfassten 3D-Oberflächen eingestellt werden.

Eine solche Maßnahme kann beispielsweise eine Warnung, die vorzugsweise auf/von der Anzeige ausgegeben wird, ein Bremsen, Stoppen, Umlenken, Unterdrücken einer Richtungskomponente der Bewegung des Roboterarms und/oder ein Hervorheben der ersten optischen Aufnahme, beispielsweise wie voranstehend beschrieben, sein.

Eine drohende Kollision (die aufgrund der erfassten 3D-Oberflächen erkannt wird) kann zwischen Elementen des Robotersystems, wie dem Roboterarm, dem Endeffektor und/oder an dem Endeffektor angebrachten Vorrichtungen, wie beispielsweise der ersten optischen Aufnahmevorrichtung oder chirurgischen Instrumenten, und anderen Objekten im Kollisionsraum des Roboters, wie beispielsweise dem Patienten, drohen und/oder erkannt werden und bevorzugt zumindest eine Maßnahme, wie voranstehend beschrieben, auslösen.

Bevorzugt weist das Robotersystem eine weitere (zweite) optische Aufnahmevorrichtung auf, die eingerichtet ist, eine weitere (zweite) optische Aufnahme zu erstellen. Bevorzugt ist die weitere (zweite) optische Aufnahmevorrichtung am Roboterarm, besonders bevorzugt am Endeffektor, angebracht. Bevorzugt ist die weitere (zweite) optische Aufnahmevorrichtung ein Mikroskop und die weitere (zweite) optische Aufnahme eine mikroskopische Aufnahme.

Eine solche Aufnahme kann einen Anwender beispielsweise bei einer Operation unterstützen, indem das Operationsgebiet (Körperteil/ Körperstelle) (vergrößert) aufgenommen wird/ werden kann und/oder darstellbar ist/ dargestellt wird.

Bevorzugt ist (dazu) die zweite optische Aufnahmevorrichtung auf das Operationsgebiet ausgerichtet und/oder fokussiert. Das heißt, bevorzugt ist die zweite optische Aufnahmevorrichtung zum Beispiel auf ein zu operierendes Körperteil ausgerichtet und/oder fokussiert. Die Offenbarung ist aber nicht darauf beschränkt. Insbesondere bei Einsätzen des medizinischen Robotersystems in anderen medizinischen, nicht-operativen Fachbereichen kann beispielsweise die zweite optische Aufnahmevorrichtung auf einen Bereich, beispielsweise einen Behandlungs-/Untersuchungsbereich, gerichtet sein und die zweite optische Aufnahme kann dann entsprechend eine Aufnahme von diesem Bereich sein.

Bevorzugt ist die Anzeige eingerichtet, (wahlweise bzw. abhängig von der Roboterarmbewegung) die erste und zweite optische Aufnahme, gemeinsam miteinander (und/oder gleichzeitig) und ohne die jeweils andere abzubilden. Wenn die erste und die zweite optische Aufnahme gemeinsam (und/oder gleichzeitig) auf der Anzeige abgebildet werden, kann ein Anwender darauf bevorzugt mit der Abbildung der ersten optischen Aufnahme die Gesamtszenerie (zum Beispiel Operationstisch, Patient usw.) und gleichzeitig mit der Abbildung der zweiten optischen Aufnahme zum Beispiel das Operationsgebiet (detaillierter) sehen. Bevorzugt ist die Anzeige ein Monitor oder Bildschirm, sie kann aber auch mehrere Monitore oder Bildschirme aufweisen. Bevorzugt wird die erste optische Aufnahme auf demselben Monitor oder Bildschirm abgebildet, wie die zweite optische Aufnahme. Dies hat dann den Vorteil, dass beispielsweise ein Anwender, der aufgrund der zweiten optischen Aufnahme (bereits) auf die Anzeige schaut, dann das Abbilden der ersten optischen Aufnahme bemerkt und beide Aufnahmen auch gleichzeitig/ gemeinsam wahrnehmen kann. Die Offenbarung ist aber nicht darauf beschränkt, die erste optische Aufnahme und die zweite optische Aufnahme können auch (gleichzeitig) auf verschiedenen Monitoren oder Bildschirmen angezeigt werden.

Bevorzugt ist das Robotersystem und/oder die Anzeige und/oder die zweite optische Aufnahmevorrichtung eingerichtet, dass die zweite optische Aufnahme als Liveübertragung bzw. Livestream bzw. Echtzeitübertragung auf der Anzeige abgebildet wird/ abbildbar ist. In anderen Worten wird also bevorzugt, wenn die Anzeige die zweite optische Aufnahme anzeigt, das angezeigt, was zu (nahezu) diesem Zeitpunkt die erste optische Aufnahmevorrichtung sieht und/oder als zweite optische Aufnahme aufnimmt.

Die Anzeige kann eingerichtet sein, bei einem gemeinsamen Abbilden der ersten optischen Aufnahme und der zweiten optischen Aufnahme, die erste oder die zweite optische Aufnahme (stärker) hervorzuheben, vorzugsweise wie voranstehend beschrieben. Bevorzugt ist die Anzeige eingerichtet, bei einem gemeinsamen Abbilden die erste optische Aufnahme kleiner als die zweite optische Aufnahme abzubilden. Vorzugsweise wird die erste optische Aufnahme in der zweiten optischen Aufnahme abgebildet. Bevorzugt wird also die erste optische Aufnahme in der zweiten optischen Aufnahme sozusagen als Bild-im-Bild bzw. im Vordergrund abgebildet. Dies kann ggfls. auf die erste optische Aufnahme aufmerksam machen und dabei kann die zweite optische, vorzugsweise mikroskopische, Aufnahme größer/ groß genug abgebildet werden, damit zum Beispiel das Operationsgebiet klar zu erkennen ist.

Die Anzeige kann eingerichtet sein, insbesondere während einer Bewegung des Roboterarms, die erste optische Aufnahme hervorgehoben auf der Anzeige abzubilden, vorzugsweise wie voranstehend beschrieben, beispielsweise auch stärker als die zweite optische Aufnahme. Es kann also zum Beispiel, bevorzugt während einer Bewegung des Roboterarms, die erste optische Aufnahme größer (als bei Stillstand des Roboterarms) und/oder zum Beispiel mit einer Markierung, Skala oder Warnung abgebildet werden. Die Anzeige kann auch eingerichtet sein, die erste optische Aufnahme in Abhängigkeit einer Bewegung des Roboterarms, vorzugsweise während der Bewegung, ohne die zweite optische Aufnahme/ anstelle der zweiten optischen Aufnahme abzubilden und/oder (nur, sonst) bei Bewegungslosigkeit des Roboterarms die zweite optische Aufnahme abzubilden. Es kann auch in Abhängigkeit einer Bewegung des Roboterarms die erste optische Aufnahme auf einem ersten (Haupt-) Bildschirm der Anzeige abgebildet werden (auf dem sonst beispielsweise die zweite optische Aufnahme abgebildet wird) und die zweite optische Aufnahme kann dann auf einem zweiten Bildschirm abgebildet werden.

Eine/ Die Bewegung des Roboters und/oder Roboterarms kann beispielsweise anhand einer Änderung eines Gelenkwinkels eines Gelenks des Roboterarms, mit Navigationsmarkern am Roboterarm, anhand eines Steuersignals/ Bewegungsbefehls an den Roboter und/oder Roboterarm, beispielweise über einen Joy-Stick, und/oder anhand einer (vorübergehenden) Änderung einer Perspektive von der ersten optischen Aufnahme und/oder der zweiten optischen Aufnahme bzw. Änderung der Ausrichtung der ersten und/oder zweiten optischen Aufnahmevorrichtung erkannt und/oder überprüft werden.

Bevorzugt ist die erste und/oder die zweite optische Aufnahmevorrichtung drehbar und/oder beweglich steuerbar am Roboterarm, vorzugsweise dem Endeffektor, angebracht. Das Robotersystem und/oder die erste optische Aufnahmevorrichtung und/oder die zweite optische Aufnahmevorrichtung kann eingerichtet sein, eine Gegenbewegung einer/ der jeweiligen optischen Aufnahmevorrichtung(en) durchzuführen und damit eine Bewegung des Roboterarms zu kompensieren, sodass die Ausrichtung einer/ der jeweiligen optischen Aufnahmevorrichtung(en) oder die Perspektive einer/ der jeweiligen optischen Aufnahme(n) erhalten wird. Dies kann von Vorteil sein, wenn zum Beispiel ein Anwender den Roboterarm aus dem Weg bewegen will, aber dabei die erste oder zweite optische Aufnahme möglichst nicht verändern will. Eine Bewegung des Roboterarms kann dann auch anhand einer solchen Gegenbewegung erkannt werden.

Bevorzugt verfügt die erste und/oder die zweite optische Aufnahmevorrichtung über einen Autofokus.

### Kurzbeschreibung der Figuren

Die Offenbarung wird im Folgenden anhand bevorzugter Ausführungsformen und unter Bezugnahme auf die beigefügten Figuren näher erläutert.
Fig. 1 zeigt ein medizinisches Robotersystem gemäß der vorliegenden Offenbarung;
Fig. 2 zeigt das medizinische Robotersystem aus Fig. 1 mit einem Roboterarm im Stillstand gemäß der vorliegenden Offenbarung;
Fig. 3 zeigt das medizinische Robotersystem aus Fig. 1 und 2 mit dem Roboterarm in Bewegung gemäß der vorliegenden Offenbarung;
Fig. 4 zeigt ein Ablaufdiagramm und veranschaulicht, wie ein Abbilden auf einer Anzeige gemäß der vorliegenden Offenbarung von einer Bewegung abhängen kann;
Fig. 5 zeigt ein Beispiel für ein Hervorheben einer ersten optischen Aufnahme gemäß der vorliegenden Offenbarung;
Fig. 6 zeigt ein weiteres Beispiel für ein Hervorheben einer ersten optischen Aufnahme gemäß der vorliegenden Offenbarung;
Fig. 7 zeigt ein weiteres Beispiel für ein Hervorheben einer ersten optischen Aufnahme gemäß der vorliegenden Offenbarung;
Fig. 8 zeigt ein weiteres Beispiel für ein Hervorheben einer ersten optischen Aufnahme gemäß der vorliegenden Offenbarung;
Fig. 9 zeigt ein Ablaufdiagramm und veranschaulicht, wie gemäß der vorliegenden Offenbarung beispielsweise eine drohende Kollision erkannt und darauf reagiert werden kann;
Fig. 10 veranschaulicht beispielhaft, wie eine Bewegung eines Roboterarms gemäß der vorliegenden Offenbarung erkannt werden kann.

Die Figuren sind schematisch und dienen lediglich dem Verständnis der Offenbarung. Die Merkmale der verschiedenen Ausführungsformen können untereinander ausgetauscht werden.

### Detaillierte Beschreibung der bevorzugten Ausführungsformen

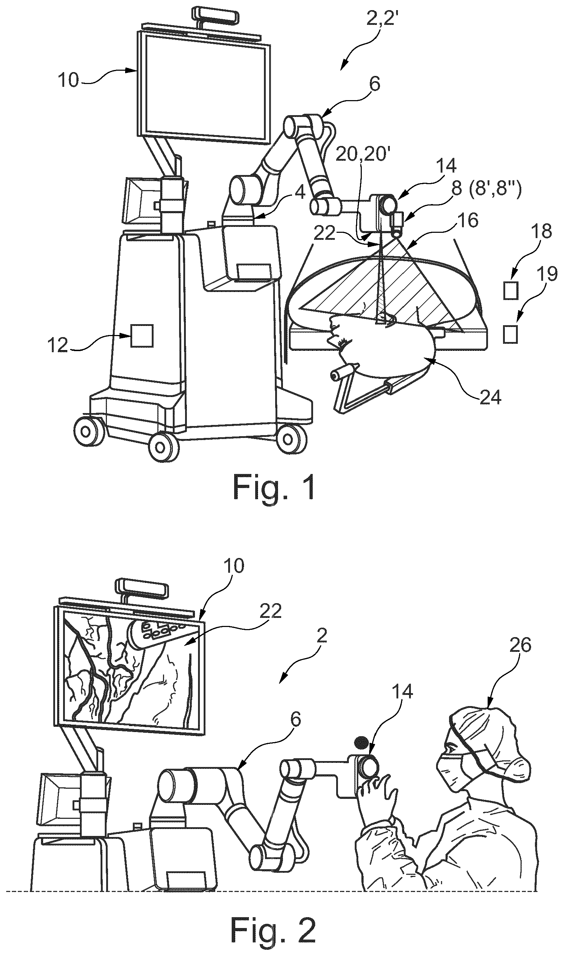

Fig. 1 zeigt ein medizinisches Robotersystem 2. In diesem Fall ist es ein chirurgisches Robotersystem. Das Robotersystem 2 weist einen Roboter 4 mit einem beweglichen Roboterarm 6, eine erste optische Aufnahmevorrichtung 8, eine Anzeige 10 und eine Auslösesteuerung 12 auf.

Der Roboterarm 6 hat einen Endeffektor 14. Die erste optische Aufnahmevorrichtung 8 ist am Roboterarm 6, in diesem Fall am Endeffektor 14, angebracht und eingerichtet, eine erste optische Aufnahme 16 zu erstellen. Die erste optische Aufnahmevorrichtung 8 kann eine 2D-Kamera 8' oder eine 3D-Kamera 8" sein. Insbesondere kann die Aufnahmevorrichtung 8 weitwinklig sein. Die erste optische Aufnahmevorrichtung 8 kann auf ein Operationsfeld bzw. einen Patienten 24 ausgerichtet sein.

Der Roboter 4 ist zum Positionieren chirurgischer Instrumente 18 und eines Visualisierungssystem 19 eingerichtet. In diesem Fall weist das Robotersystem 2 eine zweite optische Aufnahmevorrichtung 20 auf, hier ein Mikroskop 20'. Hier ist das Mikroskop 20 also ein/ das Visualisierungssystem 19. Die zweite optische Aufnahmevorrichtung 20 ist am Roboterarm, in diesem Fall ebenfalls am Endeffektor 14, angebracht. Die zweite optische Aufnahmevorrichtung 20' ist eingerichtet, eine zweite optische, in diesem Fall mikroskopische, Aufnahme 22 zu erstellen.

Die Anzeige 10 ist eingerichtet, die erste optische Aufnahme 16 abzubilden. Zudem kann die Anzeige 10 eingerichtet sein, die zweite optische Aufnahme 22 abzubilden. Beispielsweise kann die Anzeige 10 eingerichtet sein, die erste optische Aufnahme 16 und die zweite optische Aufnahme 22 jeweils ohne die andere und auch beide gemeinsam/ gleichzeitig anzuzeigen. Die zweite optische Aufnahmevorrichtung 20 kann auf den Patienten 24 ausgerichtet sein.

Die Auslösesteuerung 12 ist eingerichtet, ein Abbilden der ersten optischen Aufnahme 16 auf der Anzeige 10 in Abhängigkeit einer Bewegung des Roboterarms 6 zu steuern. Erfindungsgemäß ist die Auslösesteuerung 12 eingerichtet, das Abbilden der ersten optischen Aufnahme 16 auf der Anzeige 10 in Abhängigkeit eines Startens der Bewegung des Roboterarms 6, vorzugsweise mit Beginn der Bewegung des Roboterarms 6, zu beginnen und das Abbilden der ersten optischen Aufnahme 16 auf der Anzeige 10 in Abhängigkeit eines Stoppens der Bewegung des Roboterarms 6, vorzugsweise mit Beendigung der Bewegung des Roboterarms 6, zu beenden.

Fig. 2 zeigt das medizinische Robotersystem 2 aus Fig. 1 und einen Anwender 26 dessen, in diesem Fall einen Chirurgen. Fig. 2 zeigt den Roboterarm 6 in Ruhe/ im Stillstand, also nicht in Bewegung. Dies ist durch den Punkt über dem Endeffektor 14 veranschaulicht. Die erste optische Aufnahme 16 ist nicht auf der Anzeige 10 abgebildet. Die zweite optische Aufnahme 22 ist auf der Anzeige 10 abgebildet.

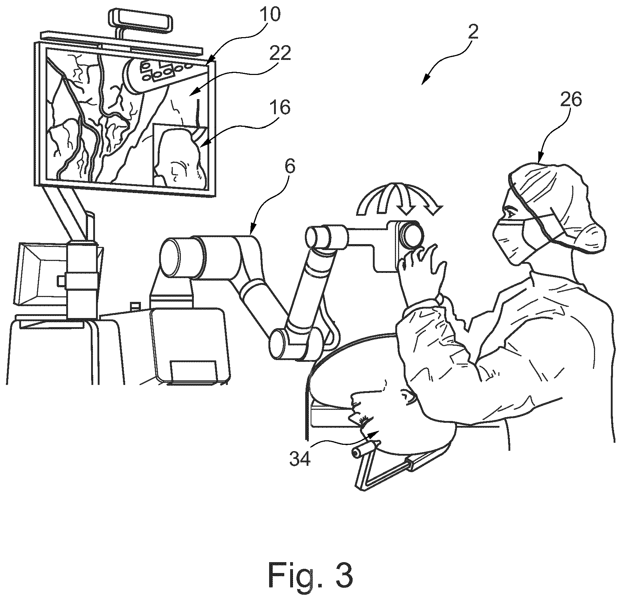

Fig. 3 zeigt das medizinische Robotersystem 2 aus Fig. 1 und Fig. 2, aber jetzt ist der Roboterarm 6 in Bewegung. Dies ist durch die Pfeile über dem Endeffektor 14 veranschaulicht. Die Anzeige 10 bildet die erste optische Aufnahme 16 ab. Zudem bildet die Anzeige 10 die zweite optische Aufnahme 22 ab. Die Abbildung der ersten optischen Aufnahme 16 wird kleiner und im Vordergrund bzw. als Bild-im-Bild, also hervorgehoben, in/innerhalb der Abbildung der zweiten optischen Aufnahme 22 abgebildet. Da die erste optische Aufnahme 16 im Vordergrund abgebildet wird, wird die Aufmerksamkeit des Anwenders 26 darauf gelenkt.

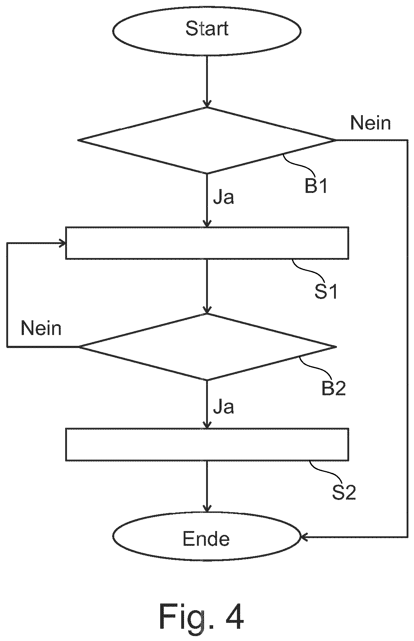

Fig. 4 zeigt ein Ablaufdiagramm zur Veranschaulichung, zu welchem Zeitpunkt oder wie bevorzugt die Anzeige 10 die erste optische Aufnahme 16 und/oder die zweite optische Aufnahme 22 anzeigt. Der gezeigte Ablauf wird beispielsweise von dem Robotersystem 2 aus Fig. 1 durchgeführt. Zunächst wird überprüft, ob der Roboterarm 6 eine Bewegung beginnt. Wenn der Roboterarm 6 eine Bewegung beginnt, ist die Bedingung B1 erfüllt (Ja). Wenn der Roboterarm 6 keine Bewegung beginnt bzw. die Bedingung B1 nicht erfüllt ist (Nein), wird der Ablauf beendet. Beginnt der Roboterarm eine Bewegung, wird in Schritt S1 das Abbilden der ersten optischen Aufnahme 16 auf der Anzeige 10 begonnen bzw. die erste optische Aufnahme 16 auf der Anzeige 10 abgebildet, wie es beispielsweise in Fig. 3 gezeigt ist.

Es wird dann in der Bedingung B2 überprüft, ob die Bewegung des Roboterarms 6 beendet wird. Die Bedingung B2 ist erfüllt (Ja), wenn die Bewegung des Roboterarms 6 beendet wird. Wenn die Bewegung des Roboterarms 6 nicht beendet wird bzw. die Bedingung B2 nicht erfüllt ist (Nein), wird der Ablauf in Schritt S1 fortgeführt, und die erste optische Aufnahme 16 (weiterhin) abgebildet. Wird die Bewegung des Roboterarms 6 beendet bzw. ist die Bedingung B2 erfüllt (Ja), so wird in Schritt S2 das Abbilden der ersten optischen Aufnahme 16 auf der Anzeige 10 beendet bzw. die erste optische Aufnahme 16 nicht (mehr) auf der Anzeige 10 abgebildet (vgl. Fig. 2). Danach wird der Ablauf beendet. Der Ablauf wird vorzugsweise wiederholt/ dauerhaft, beispielsweise wenn das Robotersystem 2 eingeschalten ist, ausgeführt.

Alternativ kann/ wird in der Bedingung B1 überprüft (werden), ob sich der Roboterarm 6 in Bewegung befindet. Entsprechend wird dann die erste optische Aufnahme 16 abgebildet und in B2 überprüft, ob sich der Roboterarm 6 nicht (mehr) in Bewegung befindet.

Alternativ oder zusätzlich kann/ wird in Schritt S1 die erste optische Aufnahme 16 hervorgehoben oder ein Hervorheben begonnen (werden). Entsprechend kann/ wird in S2 die erste optische Aufnahme 16 nicht (mehr) hervorgehoben (werden) oder das Hervorheben beendet (werden).

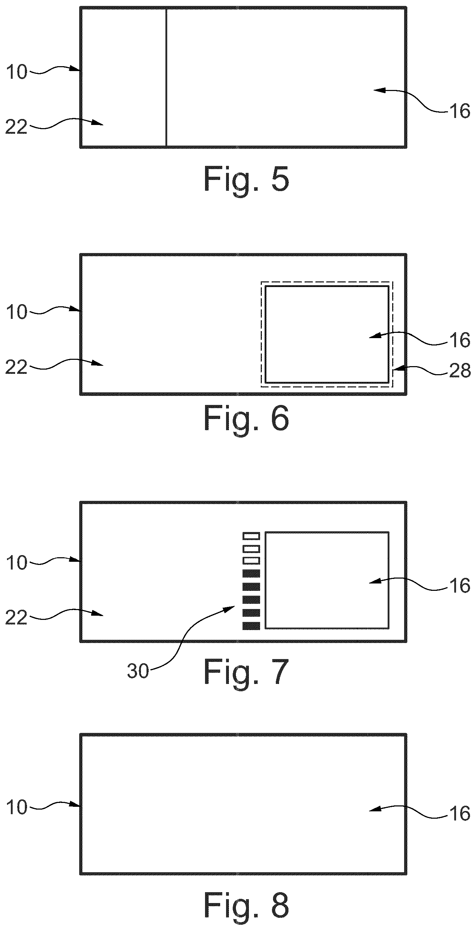

Fig. 5 zeigt ein Beispiel für ein Hervorheben einer ersten optischen Aufnahme 16, die der aus Fig. 3 entspricht. Die erste optische Aufnahme 16 wird (bei Bewegung des Roboterarms 6) hervorgehoben, indem diese auf einem Großteil einer (der Anzeige 10 aus Fig. 1 entsprechenden) Anzeige 10 dargestellt wird. Die Anzeige 10 zeigt insbesondere die erste optische Aufnahme 16 größer als die zweite optische Aufnahme 22 an.

Fig. 6 zeigt ein weiteres Beispiel zu Fig. 5 für ein Hervorheben der ersten optischen Aufnahme 16. Die erste optische Aufnahme 16 wird (bei Bewegung des Roboterarms 6) hervorgehoben, indem die erste optische Aufnahme 16 mit einer Markierung 28 abgebildet bzw. umrandet wird.

Fig. 7 zeigt ein weiteres Beispiel für ein Hervorheben der ersten optischen Aufnahme 16. Die erste optische Aufnahme 16 wird (bei Bewegung des Roboterarms 6) hervorgehoben, indem die erste optische Aufnahme 16 mit einer Skala 30 versehen wird. Die Skala 30 kann beispielsweise eine Geschwindigkeit einer Bewegung des Roboterarms 6 angeben/ visualisieren.

Fig. 8 zeigt ein weiteres Beispiel für ein Hervorheben der ersten optischen Aufnahme 16. Die erste optische Aufnahme 16 wird (bei Bewegung des Roboterarms 6) hervorgehoben, indem die erste optische Aufnahme 16 alleine (insbesondere ohne die zweite optische Aufnahme 22) auf der Anzeige 10 abgebildet wird.

Ein weiteres, bevorzugtes Beispiel für ein Hervorheben der ersten optischen Aufnahme 16 ist in Fig. 3 gezeigt. Die erste optische Aufnahme 16 wird (bei Bewegung des Roboterarms 6) hervorgehoben, indem die erste optische Aufnahme 16 im Vordergrund (der zweiten optische Aufnahme 22) bzw. als Bild-im-Bild abgebildet wird.

Die Anzeige 10 ist eingerichtet, die erste optische Aufnahme 16 und/oder die zweite optische Aufnahme 22 auf die gezeigten Weisen abzubilden.

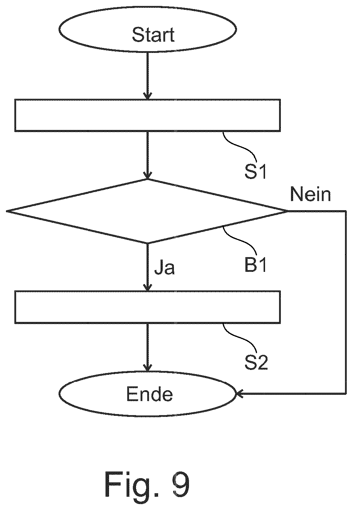

Fig. 9 zeigt ein Ablaufdiagramm und veranschaulicht, wie gemäß der vorliegenden Offenbarung beispielsweise eine drohende Kollision 32 erkannt und darauf reagiert bzw. welche Maßnahme ergriffen werden kann. Der gezeigte Ablauf wird beispielsweise von dem Robotersystem 2 aus Fig. 1 ausgeführt. In Schritt S1 erfasst die erste optische Aufnahmevorrichtung 8 3D-Oberflächen 34, beispielsweise eines Patienten 24, wie in Fig. 3 dargestellt. Die 3D-Oberflächen 34 werden beispielsweise mit Stereo-Rekonstruktion, Punktwolken und/oder Lidar erfasst/ erhalten. Dies kann zum Beispiel vor oder während einer Bewegung des Roboterarms 6 passieren. Anschließend wird, beispielsweise während/ für eine Bewegung des Roboterarms 6 die Bedingung B1 überprüft. Dabei wird anhand der erfassten 3D-Oberflächen 34 überprüft, ob es eine drohende Kollision 32 zwischen den erfassten 3D-Oberflächen 34 und beispielsweise dem sich bewegendem Roboterarm 6 oder daran angebrachten Objekten, wie beispielsweise chirurgischen Instrumenten 18, gibt. Droht eine Kollision (Ja), wird der Ablauf in S2 fortgeführt. Droht keine Kollision (Nein), wird der Ablauf beendet. In S2 wird auf die drohende Kollision 34 reagiert. Es kann/ wird eine Warnung 36 auf der Anzeige 10 des Robotersystems 2, ausgegeben (werden). Die Markierung 28 aus Fig. 6 kann/ ist so eine Warnung 36 (sein), und blinkt beispielsweise rot. Somit wird ein Anwender 26 auf die drohende Kollision 34 aufmerksam gemacht. Außerdem kann/ wird die Bewegung des Roboterarms 6 gestoppt (werden).

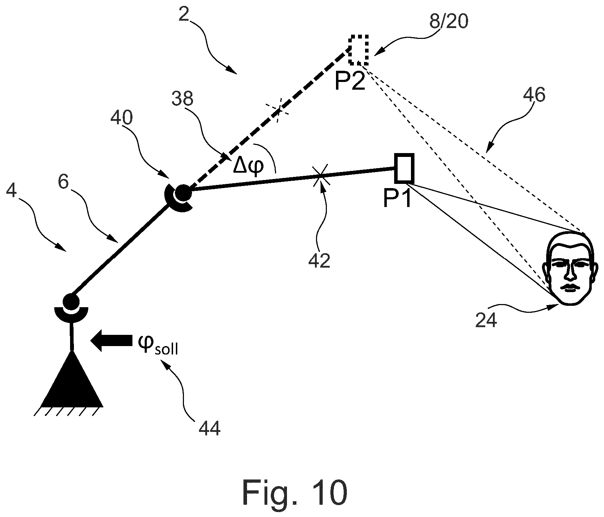

Fig. 10 zeigt, wie eine Bewegung des Roboterarms 6 eines Robotersystems 2 wie aus Fig. 1 erkannt werden kann/ wird. Das Robotersystem 2 und insbesondere der Roboter 4 ist hier vereinfacht und nur mit zwei Gliedern des Roboterarms 6 dargestellt. Der Roboterarm 6 bzw. der Endeffektor 14 bewegt sich von P1 zu P2. Die Bewegung wird anhand einer Änderung eines Gelenkwinkels 38 (Δ φ) eines Gelenks 40 des Roboterarms 6, mit Navigationsmarkern 42 am Roboterarm 6, anhand eines Steuersignals 44 (φₛₒₗₗ) an den Roboter 4 oder Roboterarm 6, und/oder anhand einer Änderung einer Perspektive 46 von der ersten optischen Aufnahme 16 und/oder der zweiten optischen Aufnahme 22 erkannt.

### Liste der Bezugszeichen

- 2: medizinisches Robotersystem
- 4: Roboter
- 6: Roboterarm
- 8: erste optische Aufnahmevorrichtung
- 8': 2D-Kamera
- 8": 3D-Kamera
- 10: Anzeige
- 12: Auslösesteuerung
- 14: Endeffektor
- 16: erste optische Aufnahme
- 18: chirurgische Instrumente
- 19: Visualisierungssystem
- 20: zweite optische Aufnahmevorrichtung
- 20': Mikroskop
- 22: zweite optische Aufnahme
- 24: Patient
- 26: Anwender
- 28: Markierung
- 30: Skala
- 32: drohende Kollision
- 34: 3D-Oberfläche
- 36: Warnung
- 38: Änderung eines Gelenkwinkels
- 40: (Roboter-) Gelenk
- 42: Navigationsmarker
- 44: Steuersignal
- 46: Änderung einer Perspektive

## Patentansprüche

1. Medizinisches, vorzugsweise chirurgisches, Robotersystem (2), mit
einem Roboter (4), der zumindest einen beweglichen Roboterarm (6), vorzugsweise zum Positionieren chirurgischer Instrumente (18) und/oder eines Visualisierungssystems (19), aufweist,
einer optischen Aufnahmevorrichtung (8), die an dem Roboterarm (6), vorzugsweise einem Endeffektor (14) des Roboterarms (6), angebracht und eingerichtet ist, eine vorzugsweise weitwinklige, optische Aufnahme (16) zu erstellen, und
einer Anzeige (10), die eingerichtet ist, die optische Aufnahme (16) abzubilden,
einer Auslösesteuerung (12), die eingerichtet ist, ein Abbilden der optischen Aufnahme (16) auf der Anzeige (10) in Abhängigkeit einer Bewegung des Roboterarms (6) zu steuern, um einen Anwender (26) auf die Bewegung des Roboterarms (6) aufmerksam zu machen,
**dadurch gekennzeichnet, dass** die Auslösesteuerung (12) eingerichtet ist,
das Abbilden der optischen Aufnahme (16) auf der Anzeige (10) in Abhängigkeit eines Startens der Bewegung des Roboterarms (6), vorzugsweise mit Beginn der Bewegung des Roboterarms (6), zu beginnen und
das Abbilden der optischen Aufnahme (16) auf der Anzeige (10) in Abhängigkeit eines Stoppens der Bewegung des Roboterarms (6), vorzugsweise mit Beendigung der Bewegung des Roboterarms (6), zu beenden.

2. Medizinisches Robotersystem (2) nach Anspruch 1, **dadurch gekennzeichnet, dass** die optische Aufnahmevorrichtung (8) eine 2D-Kamera (8') oder eine 3D-Kamera (8") ist.

3. Medizinisches Robotersystem (2) nach einem der Ansprüche 1 oder 2, **dadurch gekennzeichnet, dass** die optische Aufnahmevorrichtung (8) eingerichtet ist, 3D-Oberflächen (34), vorzugsweise mit Stereo-Rekonstruktion, Punktwolken und/oder Lidar, zu erfassen.

4. Medizinisches Robotersystem (2) nach Anspruch 3, **dadurch gekennzeichnet, dass** das Robotersystem (2) eingerichtet ist, aufgrund der erfassten 3D-Oberflächen (34) eine drohende Kollision (32) mit dem Robotersystem (2) zu erkennen und/oder eine Warnung (36), vorzugsweise auf der Anzeige (10), auszugeben und/oder die Bewegung des Roboterarms (6) zu stoppen.

5. Medizinisches Robotersystem (2) nach einem der Ansprüche 1 bis 4, **dadurch gekennzeichnet, dass** das Robotersystem (2) eine weitere optische Aufnahmevorrichtung (20), vorzugsweise ein Mikroskop (20'), aufweist, die eingerichtet ist, eine weitere optische, vorzugsweise mikroskopische, Aufnahme (22) zu erstellen.

6. Medizinisches Robotersystem (2) nach Anspruch 5, **dadurch gekennzeichnet, dass** die Anzeige (10) eingerichtet ist, die weitere optische Aufnahme (22) gemeinsam mit und ohne die optische Aufnahme (16) abzubilden.

7. Medizinisches Robotersystem (2) nach Anspruch 6, **dadurch gekennzeichnet, dass** die Anzeige (10) eingerichtet ist, bei einem gemeinsamen Abbilden der optischen Aufnahme (16) und der weiteren optischen Aufnahme (22) die optische Aufnahme (16) kleiner als die weitere optische Aufnahme (22), vorzugsweise die optische Aufnahme (16) in der weiteren optischen Aufnahme (22), abzubilden.

8. Medizinisches Robotersystem (2) nach einem der Ansprüche 1 bis 7, **dadurch gekennzeichnet, dass** die Bewegung des Roboterarms (6) anhand einer Änderung eines Gelenkwinkels (38) eines Gelenks (40) des Roboterarms (6), mit Navigationsmarkern (42) am Roboterarm (6), anhand eines Steuersignals (44) an den Roboter (4) und/oder Roboterarm (6), und/oder anhand einer Änderung einer Perspektive (46) von der optischen Aufnahme (16) erkannt wird.

## Claims

1. A medical, preferably surgical, robot system (2), comprising
a robot (4) which has at least one movable robot arm (6), preferably for positioning surgical instruments (18) and/or a visualization system (19),
an optical recording device (8), which is attached to the robot arm (6), preferably to an end effector (14) of the robot arm (6), and is designed to generate a preferably wide-angle optical recording (16), and
a display (10), which is designed to depict the optical recording (16),
a trigger control (12) which is adapted to control imaging of the optical recording (16) on the display (10) depending on a movement of the robot arm (6) in order to make a user (26) aware of the movement of the robot arm (6),
**characterized in that** the trigger control (12) is configured to
begin imaging of the optical recording (16) on the display (10) based on the start of a movement of the robot arm (6), preferably upon the beginning of the movement of the robot arm (6), and
terminate imaging of the optical recording (16) on the display (10) based on the stopping of the movement of the robot arm (6), preferably at the end of the movement of the robot arm (6).

2. The medical robot system (2) according to claim 1, **characterized in that** the optical recording device (8) is a 2D camera (8') or a 3D camera (8").

3. The medical robot system (2) according to any of claims 1 or 2, **characterized in that** the optical recording device (8) is designed to capture 3D surfaces (34), preferably with stereo reconstruction, point clouds, and/or lidar.

4. The medical robot system (2) according to claim 3, **characterized in that** the robot system (2) is adapted to detect an impending collision (32) with the robot system (2) based on the detected 3D surfaces (34) and/or to issue a warning (36), preferably on the display (10), and/or to stop the movement of the robot arm (6).

5. The medical robot system (2) according to any of claims 1 to 4, **characterized in that** the robot system (2) has a further optical recording device (20), preferably a microscope (20'), which is adapted to create a further optical, preferably microscopic, recording (22).

6. The medical robot system (2) according to claim 5, **characterized in that** the display (10) is adapted to display the additional optical recording (22) together with and without the optical recording (16).

7. The medical robot system (2) according to claim 6, **characterized in that** the display (10) is configured to display the optical recording (16) smaller than the additional optical recording (22), preferably the optical recording (16) within the additional optical recording (22), when imaging the optical recording (16) and the additional optical recording (22) together.

8. The medical robot system (2) according to any of claims 1 to 7, **characterized in that** the movement of the robot arm (6) is recognized by means of a change in the joint angle (38) of a joint (40) of the robot arm (6), by navigation markers (42) on the robot arm (6), on the basis of a control signal (44) to the robot (4) and/or robot arm (6), and/or by means of a change in perspective (46) from the optical recording (16).

## Revendications

1. Système de robot (2) médical, de préférence chirurgical avec
un robot (4) qui présente au moins un bras de robot (6) mobile, de préférence pour le positionnement d'instruments chirurgicaux (18) et/ou d'un système de visualisation (19),
un dispositif d'enregistrement optique (8) qui est monté au niveau du bras de robot (6), de préférence un effecteur d'extrémité (14) du bras de robot (6) et conçu afin de générer un enregistrement optique de préférence à grand angle et
un écran (10) qui est conçu afin d'illustrer l'enregistrement optique (16),
une commande de déclenchement (12) qui est conçue afin de commander une illustration de l'enregistrement optique (16) sur l'écran (10) en fonction d'un mouvement du bras de robot (6), afin d'attirer l'attention d'un utilisateur (26) sur le mouvement du bras de robot (6),
**caractérisé en ce que** la commande de déclenchement (12) est conçue
afin de commencer l'illustration de l'enregistrement optique (16) sur l'écran (10) en fonction d'un démarrage du mouvement du bras de robot (6), de préférence au début du mouvement du bras de robot (6) et
afin de terminer l'illustration de l'enregistrement optique (16) sur l'écran (10) en fonction d'un arrêt du mouvement du bras de robot (6), de préférence à la fin du mouvement du bras de robot (6).

2. Système de robot (2) médical selon la revendication 1, **caractérisé en ce que** le dispositif d'enregistrement optique (8) est une caméra 2D (8') ou une caméra 3D (8").

3. Système de robot (2) médical selon l'une quelconque des revendications 1 ou 2, **caractérisé en ce que** le dispositif d'enregistrement optique (8) est conçu afin de détecter des surfaces 3D (34), de préférence avec une reconstruction stéréo, des nuages de point et/ou un lidar.

4. Système de robot (2) médical selon la revendication 3, **caractérisé en ce que** le système de robot (2) est conçu afin de reconnaître, sur la base des surfaces 3D détectées (34), un risque de collision (32) avec le système de robot (2) et/ou d'émettre un avertissement (36), de préférence sur l'écran (10), et/ou d'arrêter le mouvement du bras de robot (6).

5. Système de robot (2) médical selon l'une quelconque des revendications 1 à 4, **caractérisé en ce que** le système de robot (2) présente un autre dispositif d'enregistrement optique (20), de préférence un microscope (20') qui est conçu afin d'établir un autre enregistrement (22) optique, de préférence microscopique.

6. Système de robot (2) médical selon la revendication 5, **caractérisé en ce que** l'écran (10) est conçu afin d'illustrer l'autre enregistrement optique (22) conjointement avec et sans l'enregistrement optique (16).

7. Système de robot (2) médical selon la revendication 6, **caractérisé en ce que** l'écran (10) est conçu afin d'illustrer, lors d'une illustration commune de l'enregistrement optique (16) et de l'autre enregistrement optique (22), l'enregistrement optique (16) comme étant plus petit que l'autre enregistrement optique (22), de préférence l'enregistrement optique (16) dans l'autre enregistrement optique (22).

8. Système de robot (2) médical selon l'une quelconque des revendications 1 à 7, **caractérisé en ce que** le mouvement du bras de robot (6) est reconnu à l'aide d'une modification d'un angle d'articulation (38) d'une articulation (40) du bras de robot (6), avec des marqueurs de navigation (42) au niveau du bras de robot (6), à l'aide d'un signal de commande (44) au niveau du robot (4) et/ou du bras de robot (6) et/ou à l'aide d'une modification d'une perspective (46) de l'enregistrement optique (16).
